# EUROPEAN PATENT APPLICATION

(11) **EP 4 806 365 A1**
(43) Date of publication of application: **16.09.2026**
(21) Application number: 25305343.3
(22) Date of filing: 13.03.2025
(51) Int. Cl.: A61B 6/46, A61B 6/00, A61B 6/50

(54) **BALLOON EXPANSION ASSESSMENT**

(71) Applicant: Koninklijke Philips N.V., 5656 AE Eindhoven (NL)
(72) Inventor: AUVRAY, Vincent, 5656 Eindhoven (NL); ALLAIRE, Stephane, 5656 Eindhoven (NL); LOBANTSEV, Artem, 5656 Eindhoven (NL); OLIVAN BESCOS, Javier, 5656 Eindhoven (NL); ELENBAAS, Thijs, 5656 Eindhoven (NL)
(74) Representative: Philips Intellectual Property & Standards

(57) **Abstract**

The present invention relates to expanding a balloon inside a vessel. In order to provide information about any irregularity in three dimensions, a device (10) for balloon expanding assessment comprises a data input (12), a data processor (14) and an output interface (16). The data input is configured to receive a plurality of acquired X-ray images (18) of a region of interest comprising a portion of a vessel with an inserted guidewire and an inserted balloon being inflated; the X-ray images are acquired with an X-ray imaging device over a limited angular range of a small angle. The data processor is configured to compute a 3D shape-model (20) of the balloon based on the plurality of X-ray images. The output interface is configured to provide the 3D shape-model of the balloon for identification of the 3D expansion of the inflated balloon.

## Description

### FIELD OF THE INVENTION

The present invention relates to expanding a balloon inside a vessel. The present invention relates in particular to a device for balloon expanding assessment, to an imaging system for balloon expanding assessment, and a method for balloon expanding assessment.

### BACKGROUND OF THE INVENTION

An example of cardiac treatment is the use of inflatable balloons to expand the vessel after preparation of a calcified region of the cardiac vasculature. Balloon expansion can also be used during stent deployment and during post dilation. In the CathLab (catheterization laboratory), interventional cardiologists may use live X-ray to check the correct expansion and apposition of balloons. However, from a routine 2D view, a clinician can visually assess the balloon only in two dimensions and some information can go unnoticed.

### SUMMARY OF THE INVENTION

There may thus be a need to provide information about any irregularity in three dimensions.

The object of the present invention is solved by the subject-matter of the independent claims; further embodiments are incorporated in the dependent claims. It should be noted that the following described aspects of the invention apply also for the device for balloon expanding assessment, for the imaging system for balloon expanding assessment, and for the method for balloon expanding assessment.

According to the present invention, also a device for balloon expanding assessment is provided. The device comprises a data input, a data processor and an output interface. The data input is configured to receive a plurality of acquired X-ray images of a region of interest comprising a portion of a vessel with an inserted guidewire and an inserted balloon being inflated. The X-ray images are acquired with an X-ray imaging device over a limited angular range of a small angle. The data processor is configured to compute a 3D shape-model of the balloon based on the plurality of X-ray images. The output interface is configured to provide the 3D shape-model of the balloon for identification of the 3D expansion of the inflated balloon.

As an effect, information about a current state can be provided with reduced X-ray dose due to the rather small angular range of the imaging.

The limited angular range is based on the assumption that a clinically relevant deformation of the balloon always takes place across a certain deformation angle in the sense that it is visible on 2D projections from a limited angular range.

According to an example, for identification of the 3D expansion of the inflated balloon, the data processor is configured to generate 3D image data representing the balloon based on the computed model. The output interface is configured to provide the image data to a display to present an image of the 3D shape model of the balloon based on the image data for identifying the 3D expansion of the inflated balloon.

As an advantage, the user can directly see and assess the 3D expansion of the inflated balloon.

As a result, a reliable check of the 3D expansion of the balloon is provided based on an acquisition that lasts a rather short time, e.g. an additional 2-3 seconds, and possibly in low dose. The check can be completely merged into a usual workflow. The reliable check of the 3D expansion is provided with similar accuracy with much sorter acquisition time, and minimal workflow disruption, when compared e.g. with two separated biplane high dose acquisitions. The reliable check of the 3D expansion is provided with higher comfort for the clinician if the 3D rendering option is compared to the plain biplane acquisition without additional automatization.

According to an example, the data processor is configured to identify a degree of 3D expansion of the expanded balloon. The output interface is configured to provide an indicator signal if a predetermined threshold is reached by the identified degree of 3D expansion.

This provides a fast and reliable assessment of the state of the 3D expansion of the inflated balloon.

According to an example, for a computation of the 3D shape-model of the balloon, the data processor is configured to segment the balloon in the plurality of X-ray images. The data processor is configured to segment the balloon visible on the 2D image. As an option, the balloon visible on the 2D image is segmented each time an X-ray image is acquired.

According to an example, the device computes and refines the 3D balloon model iteratively. Starting from the computed 3D balloon model at that point of the computation, the data processor is configured to estimate a global motion due to breathing and/or heart beating that best aligns its projection with each of the 2D borders. The data processor is configured to finetune parameters of the 3D shape of the balloon, so that its motion compensated 2D projection over each image matches as well as possible the extracted 2D boundaries. As an option, the data processor is configured to estimate at each acquisition instant the global motion that has been imposed on the balloon.

As a result, global motion estimation and 3D model refinement is iterated a few times. The finetuning yields an improved estimation of the shape of the balloon.

According to the present invention, also an imaging system for balloon expanding assessment is provided. The system comprises an X-ray imaging device and a device for balloon expanding assessment according to one of the preceding examples. The X-ray imaging device comprises an X-ray source and an X-ray detector that are movably held to provide image data over an angular range. The X-ray imaging device is configured to acquire images over a limited angular range of maximum 30°. The X-ray imaging device is configured to acquire the X-ray images of a region of interest comprising a portion of a vessel with an inserted guidewire and an inserted balloon being inflated.

According to an example, the limited angular range of the images is pre-determined and spans a maximum angle of 30°.

This provides fast imaging with minimal dose.

As an option, the X-ray imaging device is configured to acquire the plurality of X-ray images comprising a set of 30 images or less.

This also supports fast imaging while also reducing dose for the assessment of the expansion state.

According to an example, the X-ray imaging device is configured to acquire the plurality of X-ray images in a swiveling movement of the X-ray source. The swiveling is adapted to an axis of the balloon.

As an effect, a better visibility of the correct expansion can be provided.

According to an example, the limited angular range is pre-set based on at least one of the following parameter: i) type of balloon being inflated, ii) type of stent expanded by the balloon, iii) type of vessel in which the balloon is situated, and iv) type of inflation fluid.

According to an example, the X-ray imaging device is configured to acquire the plurality of X-ray images in a scan movement. The scan movement is provided in one direction only or in two directions.

According to an example, the X-ray imaging device is configured to start and finish a procedure of the X-ray imaging device for acquiring of the plurality of X-ray images at a current working angulation of the imaging device. As an option, the X-ray imaging device is repositioned to its original position.

This provides a seamless swivel-and-back workflow of the system.

According to the present invention, also a method for balloon expanding assessment is provided. The method comprising the following steps:
- Acquiring a plurality of X-ray images of a region of interest comprising a portion of a vessel with an inserted guidewire and an inserted balloon being inflated; the X-ray images are acquired with an X-ray imaging device over a limited angular range of maximum 30°;
- Computing a 3D shape-model of the balloon based on the plurality of X-ray images; and
- Providing the 3D shape-model of the balloon for identifying the 3D expansion of the expanded balloon.

According to an aspect, a short X-ray acquisition is leveraged with a local small-angle rotation of the C-arm to determine a model of the 3D shape of the balloon. The computed model can be directly shown to the clinician, and/or serve as a basis for triggering warnings in case of irregularities.

In an option, a short acquisition is performed manually by the clinician. In another option, predetermined acquisition settings are provided with parameters that have been determined to reach a fixed compromise between duration, angulation range and X-ray dose on the one hand versus model quality on the other hand.

For example, the acquisition can be made object-dependent: The 3D model is continuously updated, and the course of the C-arm is adapted to ensure an optimal modelling (rotation around the balloon main axis).

As an advantage, the clinician is provided with information if the inflated balloon has a regular cylindrical shape. The is helpful, for example when treating a lesion, such that the clinician can check whether the balloon is well inflated. As an application, during the preparation of the lesion, the clinician can thus be ensured that every plaque or calcium has been pushed away, yielding a free and regular lumen.

As another example, during stent apposition, the clinician can be provided with as much certainty as possible that the stent is correctly deployed along the vessel from the associated balloon inflation. Identifying a local irregularity helps in avoiding an increase in future complications linked with such irregularity if not solved.

According to an aspect, the invention leverages a short X-ray acquisition with a local small-angle rotation of the C-arm to determine a model of the 3D shape of the balloon. The computed model can be directly shown to the clinician, and/or serve as a basis for triggering warnings in case of irregularities.

The field of use is basically any X-ray interventional system applied to balloon expansion related interventions.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for balloon expanding assessment.
Fig. 2 shows an example of an X-ray image of a region of interest comprising a portion of a vessel with an inserted guidewire and an inserted balloon being expanded.
Fig. 3 shows an example of a medical imaging system for balloon expanding assessment.
Fig. 4 shows basic steps of an example of a method for balloon expanding assessment.
Fig. 5 shows a further example of a workflow for assessing balloon expansion.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

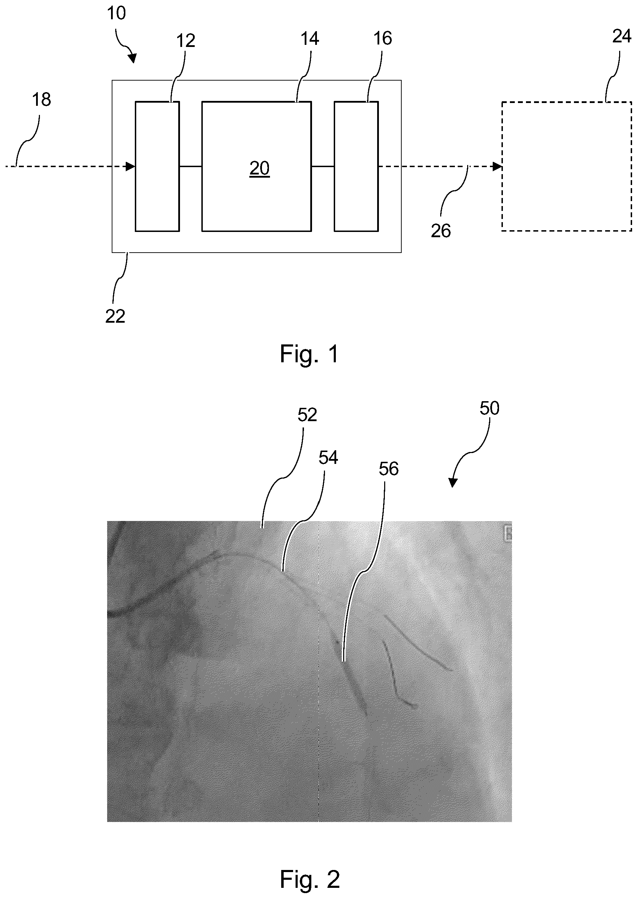

Fig. 1 schematically shows an example of a device 10 for balloon expanding assessment. The device 10 comprises a data input 12, a data processor 14 and an output interface 16. The data input 12 is configured to receive a plurality of acquired X-ray images 18 of a region of interest comprising a portion of a vessel with an inserted guidewire and an inserted balloon being inflated. The X-ray images 18 have been acquired with an X-ray imaging device over a limited angular range of a small angle. The data processor 14 is configured to compute a 3D shape-model 20 of the balloon based on the plurality of X-ray images. The output interface 16 is configured to provide the 3D shape-model 20 of the balloon for identification of the 3D expansion of the inflated balloon.

A frame 22 indicates a common enclosing or supporting structure such as a housing. The data input 12, the data processor 14 and the output interface 16 can be arranged in an integrated manner. However, in another option, the data input 12, the data processor 14 and the output interface 16 are provided separately.

As an option, the device 10 further comprises a display 24 configured to present an image of the 3D shape model of the balloon based on the image data for identifying the 3D expansion of the inflated balloon. As an option, the display 24 is also configured to display the 2D X-ray image data.

An arrow 26 indicates a data-connection of the output interface 16 and the display 24.

In an example, the X-ray images are acquired with the X-ray imaging device over a limited angular range like maximum 30°.

In an example, the output interface 16 is configured to provide the 3D shape-model of the balloon for identification of the deformation of the expanded balloon.

The term "data input" relates to providing or supplying data for data processing steps. The data input 12 can also be referred to as image data input. The data input 12 can also be referred to as data supply, as image data supply, as image input, as input unit or simply as input. In an example, the image data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the data processor 14 or controller can be provided by the output interface 16.

The final 3D model of the balloon can then be shown to the user. It can be shown as a movie of the 3D balloon rotating around its axis for instance. Or it could be a 3D-rendered view of the balloon shown from the perspective where it appears most irregular. The clinician could manipulate the 3D rending, for instance using a touchpad or a mouse.

Alternatively or additionally, the 3D shape can be analyzed to issue a warning message if some relevant pitching is detected.

The term "small angle" refers to an angular range that is far less than the usual 180⁺ degrees. A small angle is also far less than half of this, i.e. an angular range of less than 45°. For example 30° or less is provided for the angle, or even 20° or less, or 15° or less.

In an example, not shown in detail in Fig. 1, for identification of the 3D expansion of the inflated balloon, the data processor 14 is configured to generate 3D image data representing the balloon based on the computed model. The output interface 16 is configured to provide the image data to the display 24 to present an image of the 3D shape model of the balloon based on the image data for identifying the 3D expansion of the inflated balloon.

In an option, the balloon is tracked by a fluid inside the balloon used for inflation, i.e. used as a medium to apply pressure from inside the balloon.

The 3D shape-model of the balloon can be a simplified model focused on the identification of the shape and its irregularities.

The required acquisition only takes a few seconds. It can also be set to be performed in such a way that the C-arm finishes its (limited) course at its original position, thereby allowing the clinician to continue his examination without workflow adaptation. It does not need to be of exposure quality; the modelling process naturally averages out measurement noise and thus can work over noisy images.

In an example, the short acquisition is manually performed by a clinician.

In an example, not shown in detail in Fig. 1, the data processor 14 is configured to identify a degree of 3D expansion of the expanded balloon. The output interface 16 is configured to provide an indicator signal if a predetermined threshold is reached by the identified degree of 3D expansion.

In an example, the data processor 14 is configured to identify a degree of deformation of the expanded balloon. The output interface 16 is configured to provide an indicator signal if a predetermined threshold is reached by the identified degree of deformation.

In an example, not shown in detail in Fig. 1, for a computation of the 3D shape-model of the balloon, the data processor 14 is configured to segment the balloon in the plurality of X-ray images. The data processor 14 is further configured to segment the balloon visible on the 2D image, preferably each time an X-ray image is acquired.

The segmenting provides a tracking of the balloon in the images.

The segmenting of the balloon, visible on the 2D image each time an X-ray image is acquired, can be done by using a CNN network, such as a UNet, which previously has been trained for that task.

In an example, then, the balloon boundaries are delineated from the generated 2D binary map of the balloon. As the 3D geometrical model will be very sensitive to the extracted 2D boundaries, a high accuracy is needed at this step.

An example starts by initializing two contours from the coarse borders of the segmented object in the binary map, and then refines them on the original 2D image itself. For instance, the borders could be modelled as 2D splines, the control points of which would evolve in order to snap to the image gradients, with a subpixelic accuracy.

Further, the set of all previously acquired 2D images, or more exactly the extracted 2D borders associated to them, with the corresponding acquisition geometries, essentially the C-arm angles, are considered to then estimate the 3D model of the balloon.

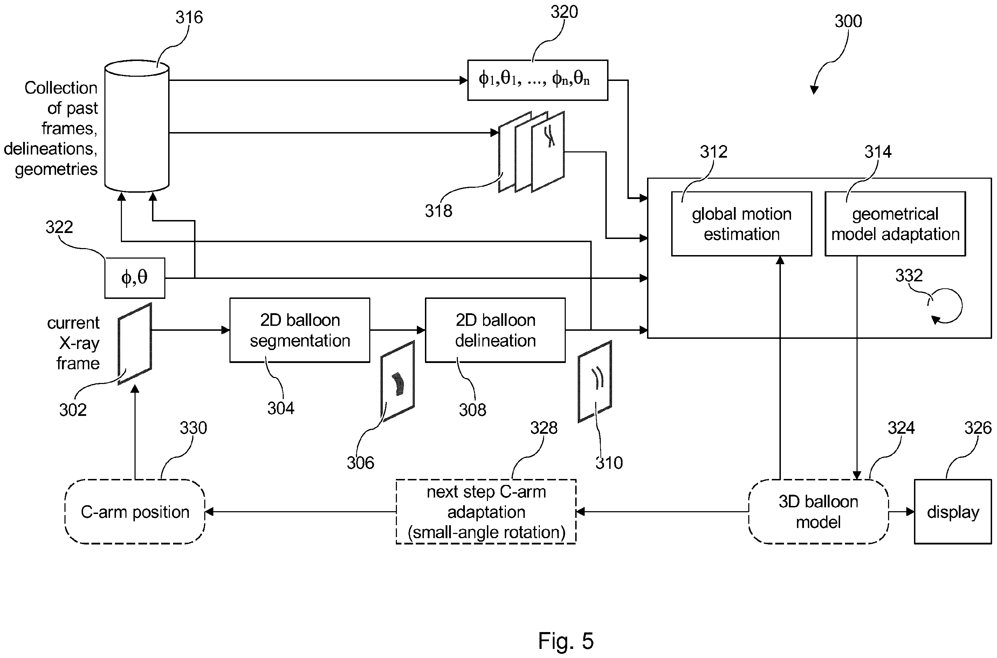

In an example, not shown in detail in Fig. 1, the device 10 computes and refines the 3D balloon model iteratively. Starting from the computed 3D balloon model at that point of the computation, the data processor 14 is configured to estimate a global motion due to breathing and/or heart beating that best aligns its projection with each of the 2D borders. The data processor 14 is further configured to finetune parameters of the 3D shape of the balloon, so that its motion compensated 2D projection over each image matches as well as possible the extracted 2D boundaries (see also Fig. 5).

As an option, the data processor 14 is configured to estimate at each acquisition instant the global motion that has been imposed on the balloon.

In an example, the estimation provides one translation per acquired 2D image.

As an option, the procedure, also referred to as framework, is repeated as long as new images are acquired. Finally, the C-arm, or any other X-ray imager, is repositioned to its original position.

In an option, the assessment is adapted to more than one balloon. The 2D work is similar, performed connected component by connected component. As an additional processing step, the different 2D balloons are paired with the 2D borders extracted from the previously acquired images. Once the additional step is performed, a global estimation and 3D model estimation step can be performed for each balloon independently.

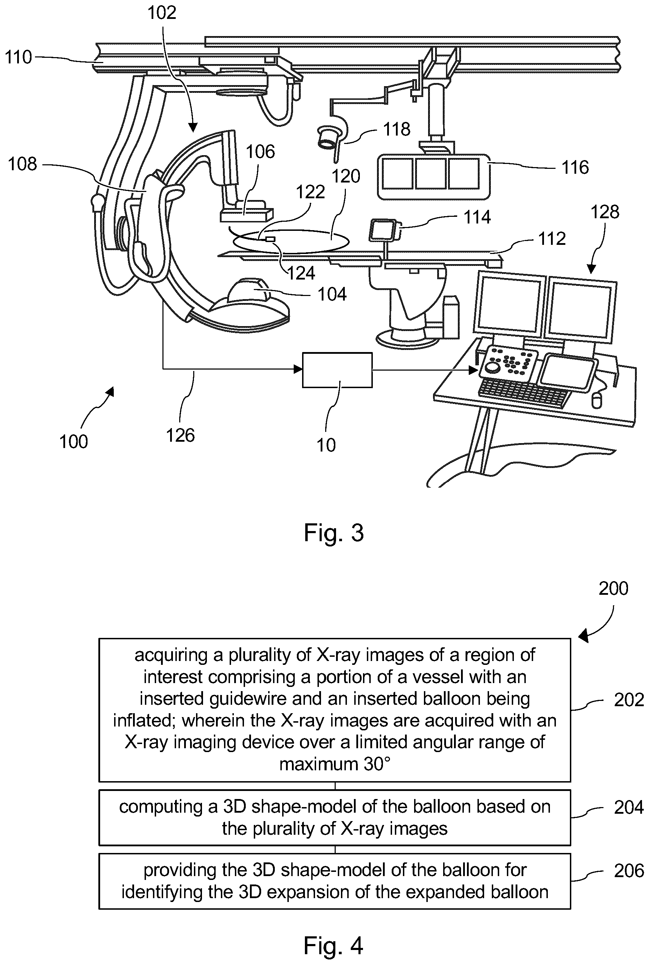

Fig. 3 shows an example of a medical imaging system 100 for balloon expanding assessment. The imaging system 100 comprises an X-ray imaging device 102 and an example of the device 10 for balloon expanding assessment according to one of the preceding and following examples. The X-ray imaging device 102 comprises an X-ray source 104 and an X-ray detector 106 that are movably held to provide image data over an angular range. The X-ray imaging device 102 is configured to acquire images over a limited angular range of maximum 30°. The X-ray imaging device 102 is configured to acquire the X-ray images of a region of interest comprising a portion of a vessel with an inserted guidewire and an inserted balloon being inflated.

Fig. 2 shows an example of an X-ray image 50 of a region of interest comprising a portion of a vessel 52 with an inserted guidewire 54 and an inserted balloon 56 being expanded. The image 50 also shows some anatomical structures. The guidewire 54 is inserted in the portion of the vessel 52. The guidewire 54 may be running in curves or even loops, following the vascular paths. The balloon is expanded. In order to better assess the expansion, a sequence of images over a small angle are acquired and image-processed as mentioned above, and also further below.

In Fig. 3, the X-ray imaging arrangement 102 is shown comprising the X-ray source 104 and the X-ray detector 106 mounted to opposing ends of a movable C-arm arrangement 108 that is suspended from a ceiling rail system 110. Further, a subject support 112 is shown with a bedside controller interface 114. Still further, a display arrangement 116 and some lighting equipment 118 can be provided. A subject 120, e.g. a patient, is arranged on the subject support 112.

In Fig. 3, as an option, the system 100 further comprises a guidewire 122 for insertion into a vascular structure of the subject. The system 100 also comprises at least one inflatable balloon 124 configured to be inserted into the vascular structure along the guidewire 122.

In Fig. 3, the guidewire 122 is inserted and the inflatable balloon 124 is inserted along the guidewire 122. Further components like an operating handle of the guidewire or a control interface for the balloon are provided, but not shown.

The X-ray imaging arrangement 102 is data-connected to the device 10 for indicating balloon expansion by a data-connection, which can be wire-bound or wireless.

As an option, Fig. 3 shows a console 128 in the right foreground that may comprise interface components like a display, keyboard, mouse, trackpad, control knobs and the like. The console is provided for operating and controlling the various equipment.

In an example of the system, the limited angular range of the images is pre-determined and spans a maximum angle of 30°.

In an option of the system, the X-ray imaging device is configured to acquire the plurality of X-ray images comprising a set of 30 images or less.

In an example, a maximum angular range of less than 30° is provided, e.g. over 25°, 20° or 15°.

In another example, a maximum angular range of 45° is provided.

In an option, one image or less is acquired for each degree of the angular range. In an example, 30 images are acquired over 30°, or less than 30 images. In another example, 15 images are acquired over 15°, or less than 15 images.

In an example of the system, the acquisition is made object-dependent.

In an option, the 3D shape-model is continuously updated, and a course of the X-ray imaging device is adapted to ensure an optimal modelling.

Criteria for an optimal modelling are, for example, suitability for segmentation of the balloon or completeness of the shape of the balloon.

In an example, after the clinician has triggered or initiated the specific acquisition, the C-arm starts performing an automatic small-angle rotation that adapts to the geometry of the balloon at hand. In a first variation, the adaptation is provided to ensure a model of higher quality with the same dose. In a second variation, the adaptation is provided to ensure a model of similar quality for a shorter acquisition, as compared to the previous two acquisition modes.

In an example of the system, the X-ray imaging device is configured to acquire the plurality of X-ray images in a swiveling movement of the X-ray source. The swiveling is adapted to the main axis of the balloon.

The swiveling movement can also be referred to as a pivoting or rotating movement.

In an example of an object-adaptive X-ray image acquisition, it is relied on a preliminary 3D model of the balloon to determine how its main axis is oriented in the 3D space. The next C-arm small-angle rotation step is computed so that it rotates around that axis. This is provided, because this rotation will give us the most information about the shape of the balloon that is of clinical interest. Hence, an intelligent and adaptative way of acquiring the images is provided giving either an improved accuracy from a similar number of images (and thus, similar dose and time), or a similar accuracy with less acquired images.

In an option, the X-ray imaging device comprises a movably hold C-arm. The X-ray source and X-ray detector are mounted to opposing ends of the C-arm.

In another option, the X-ray imaging device comprises a fist movable arm with the X-ray source, and a second movable arm with an X-ray detector. For example, the arms are robot arms.

In an example, not shown in detail in Fig. 1, the limited angular range is pre-set based on at least one of the following parameter: type of balloon being inflated, type of stent expanded by the balloon, type of vessel in which the balloon is situated and type of inflation fluid.

In an option, for computing the 3D shape-model of the balloon, it is postulated that the 3D shape of the balloon follows a given model, driven by a few parameters. For instance, it can be modelled as a 3D centerline defined by some spline nodes, that serve as centers for elliptical cross sections. In an example, the small and large axis of which can also be parametrized by some spline nodes along the centerline. In a variation, the procedural steps aim at progressively estimating the parameters, typically: a couple of dozens, that perfectly describe that 3D shape, from the observed images.

In an example, not shown in detail in Fig. 1, a movement of the X-ray imaging device for acquiring of the plurality of X-ray images is based on predetermined acquisition settings comprising at least one of the group comprising duration, angulation and X-ray radiation dose.

In an example, the acquisition settings are determined to reach a compromise between the settings (duration: preferably as short as possible; angulation: minimum range preferred; dose as little as possible) and a quality of the model to identify 3D expansion, e.g. deformation. The compromise can be fixed based on experience and user feedback.

In an example, not shown in detail in Fig. 1, the X-ray imaging device is configured to acquire the plurality of X-ray images in a scan movement. The scan movement is provided: i) in one direction only, or ii) in two directions.

In an example, the images are acquired in a first direction of the imaging device, and the imaging device is then moved back to its initial position.

In another example, the imaging device is moved to a starting position in a first direction, and the images are then acquired in a second direction of the imaging device back to its initial position.

In an example, the images are acquired in a first direction of the imaging device and in a second direction during which the device is moved back to its initial position.

In an example, not shown in detail in Fig. 1, the X-ray imaging device is configured to start and finish a procedure of the X-ray imaging device for acquiring of the plurality of X-ray images at a current working angulation of the imaging device.

In an option, the X-ray imaging device is repositioned to its original position.

In an example, a clinician initiates the acquisition, e.g. by pushing a button, and the C-arm or any other moving X-ray imaging device starts performing a predetermined rotation. In an option, the acquisition is the same, e.g. in amplitude and direction, for each patient and balloon. The C-arm is then brought back to its exact original position and angulation, thus guaranteeing that the preferred working perspective is restored, which is likely not achieved manually.

In an option, the image acquisition for the balloon modelling is performed in the continuity of the acquisition that was going on to monitor the inflation process.

Fig. 4 shows basic steps of an example of a method 200 for balloon expanding assessment. The method 200 comprises the following steps:
- In a first step 202, a plurality of X-ray images of a region of interest comprising a portion of a vessel with an inserted guidewire and an inserted balloon being inflated is acquired. The X-ray images are acquired with an X-ray imaging device over a limited angular range of maximum 30°.
- In a second step 204, a 3D shape-model of the balloon is computed based on the plurality of X-ray images.
- In a third step 206, the 3D shape-model of the balloon is provided for identifying the 3D expansion of the expanded balloon.

In an example of the method 200, for identifying the 3D expansion of the expanded balloon, it is provided the steps of: generating 3D image data representing the balloon based on the computed model; and presenting an image of the 3D shape model of the balloon based on the image data for identifying the 3D expansion of the expanded balloon.

In an example of the method, for computing the 3D shape-model of the balloon, the balloon is segmented in the plurality of X-ray images. In an option, each time an X-ray image is acquired, the balloon visible on the 2D image is segmented.

In an example of the method, starting from the computed 3D balloon model, a global motion due to breathing and/or heart beating is estimated that best aligns its projection with each of the 2D borders. Further, parameters of the 3D shape of the balloon are finetuned, so that its motion compensated 2D projection over each image matches as well as possible the extracted 2D boundaries. As an option, the global motion that has been imposed on the balloon is estimated at each acquisition instant.

Fig. 5 shows a further example of a workflow 300 for assessing balloon expansion. A current X-ray frame 302 (of a sequence over a small angular range) is provided to 2D balloon segmentation 304, resulting in segmented balloon image 306. This is provided to 2D balloon delineation 308, resulting in delineated balloon image 310. This image is then provided to a further data processing with a global motion estimation 312 and geometrical model adaptation 314. However, in this procedure also a collection 316 of past frames, delineations and geometries can be used. Previous sequences 318, as well as angular information 320 is provided to the global motion estimation 312 and the geometrical model adaptation 314. Further, also angular information 322 of the current X-ray frame is provided to the global motion estimation 312 and the geometrical model adaptation 314. The geometrical model adaptation 314 provides a 3D balloon model 324 as an option, for display 326 or for providing to the global motion estimation 312. In a further option, next step C-arm adaptation in the small-angle rotation 328 is provided, leading to C-arm position 330 for another acquisition of a new current X-ray frame. A repetition arrow 332 indicates the repetition of the steps.

In an example, a computer program or program element for controlling an apparatus according to one of the examples above is provided, which program or program element, when being executed by a processing unit, is adapted to perform the method steps of one of the method examples above.

In an example, a computer readable medium having stored the computer program of the preceding examples is provided.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. **In** particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

**In** the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for balloon expanding assessment, comprising:
- a data input (12);
- a data processor (14); and
- an output interface (16);
wherein the data input is configured to receive a plurality of acquired X-ray images (18) of a region of interest comprising a portion of a vessel with an inserted guidewire and an inserted balloon being inflated; wherein the X-ray images are acquired with an X-ray imaging device over a limited angular range of a small angle;
wherein the data processor is configured to compute a 3D shape-model (20) of the balloon based on the plurality of X-ray images; and
wherein the output interface is configured to provide the 3D shape-model of the balloon for identification of the 3D expansion of the inflated balloon.

2. Device according to claim 1, wherein, for identification of the 3D expansion of the inflated balloon, the data processor is configured to generate 3D image data representing the balloon based on the computed model; and
wherein the output interface is configured to provide the image data to a display to present an image of the 3D shape model of the balloon based on the image data for identifying the 3D expansion of the inflated balloon.

3. Device according to claim 1 or 2, wherein the data processor is configured to identify a degree of 3D expansion of the expanded balloon; and
wherein the output interface is configured to provide an indicator signal if a predetermined threshold is reached by the identified degree of 3D expansion.

4. Device according to claim 1, 2 or 3, wherein for a computation of the 3D shape-model of the balloon, the data processor is configured to segment the balloon in the plurality of X-ray images; and
wherein the data processor is configured to segment the balloon visible on the 2D image, preferably each time an X-ray image is acquired.

5. Device according to one of the preceding claims that computes and refines the 3D balloon model iteratively, wherein, starting from the computed 3D balloon model at that point of the computation, the data processor is configured to estimate a global motion due to breathing and/or heart beating that best aligns its projection with each of the 2D borders;
wherein the data processor is configured to finetune parameters of the 3D shape of the balloon, so that its motion compensated 2D projection over each image matches as well as possible the extracted 2D boundaries; and
wherein, preferably, the data processor is configured to estimate at each acquisition instant the global motion that has been imposed on the balloon.

6. A medical imaging system (100) for balloon expanding assessment, the system comprising:
- an X-ray imaging device (102); and
- a device (10) for balloon expanding assessment according to one of the preceding claims;
wherein the X-ray imaging device comprises an X-ray source (104) and an X-ray detector (106) that are movably hold to provide image data over an angular range;
wherein the X-ray imaging device is configured to acquire images over a limited angular range of maximum 30°; and
wherein the X-ray imaging device is configured to acquire the X-ray images of a region of interest comprising a portion of a vessel with an inserted guidewire and an inserted balloon being inflated.

7. System according to claim 6, wherein the limited angular range of the images is predetermined and spans a maximum angle of 30°; and
wherein, preferably, the X-ray imaging device is configured to acquire the plurality of X-ray images comprising a set of 30 images or less.

8. System according to claim 6 or 7, wherein the acquisition is made object-dependent; and
wherein, preferably, the 3D shape-model is continuously updated, and a course of the X-ray imaging device is adapted to ensure an optimal modelling.

9. System according to claim 6, 7 or 8, wherein the X-ray imaging device is configured to acquire the plurality of X-ray images in a swiveling movement of the X-ray source; and
wherein the swiveling is adapted to an axis of the balloon.

10. System according to one of claims 6 to 9, wherein the limited angular range is pre-set based on at least one of the following parameter:
i) type of balloon being inflated;
ii) type of stent expanded by the balloon;
iii) type of vessel in which the balloon is situated; and
iv) type of inflation fluid.

11. System according to one of claims 6 to 10, wherein a movement of the X-ray imaging device for acquiring of the plurality of X-ray images is based on predetermined acquisition settings comprising at least one of the group comprising duration, angulation and X-ray radiation dose.

12. System according to one of claims 6 to 11, wherein the X-ray imaging device is configured to acquire the plurality of X-ray images in a scan movement; and
wherein the scan movement is provided:
i) in one direction only; or
ii) in two directions.

13. System according to one of claims 6 to 11, wherein the X-ray imaging device is configured to start and finish a procedure of the X-ray imaging device for acquiring of the plurality of X-ray images at a current working angulation of the imaging device; and
wherein, preferably, the X-ray imaging device is repositioned to its original position.

14. A method (200) for balloon expanding assessment, the method comprising the following steps:
- acquiring (202) a plurality of X-ray images of a region of interest comprising a portion of a vessel with an inserted guidewire and an inserted balloon being inflated; wherein the X-ray images are acquired with an X-ray imaging device over a limited angular range of maximum 30°;
- computing (204) a 3D shape-model of the balloon based on the plurality of X-ray images; and
- providing (206) the 3D shape-model of the balloon for identifying the 3D expansion of the expanded balloon.

15. Computer program comprising instructions which, when the program is executed by a computer, cause the computer to carry out the method of claim 14.
